**Europäisches Patentamt**

**European Patent Office**

**Office européen des brevets**

(19)

(11) Veröffentlichungsnummer: **0 326 701 B1**

(12) **EUROPÄISCHE PATENTSCHRIFT**

(45) Veröffentlichungstag der Patentschrift: **08.07.92**

(51) Int. Cl.⁵: **A61B 17/22**

(21) Anmeldenummer: **88121348.2**

(22) Anmeldetag: **21.12.88**

(54) **Piezoelektrische Stosswellenquelle.**

(30) Priorität: **04.02.88 DE 3803275**

(43) Veröffentlichungstag der Anmeldung:
**09.08.89 Patentblatt 89/32**

(45) Bekanntmachung des Hinweises auf die
Patenterteilung:
**08.07.92 Patentblatt 92/28**

(84) Benannte Vertragsstaaten:
**CH DE ES FR IT LI**

(56) Entgegenhaltungen:
**EP-A- 0 240 797**
**FR-A- 2 567 394**
**GB-A- 2 140 693**

(73) Patentinhaber: **DORNIER MEDIZINTECHNIK
GMBH
Postfach 1128
W-8034 Germering 1(DE)**

(72) Erfinder: **Eizenhöfer, Harald, Dipl.-Phys.
Oberafferbacher Strasse 2
W-8752 Johannesberg(DE)**
Erfinder: **Marlinghaus, Ernst, Dr.rer.nat.
Friedlandstrasse 7
W-8034 Germering(DE)**

(74) Vertreter: **Landsmann, Ralf, Dipl.-Ing.
DORNIER GMBH - Patentabteilung - Kleeweg
3
W-7990 Friedrichshafen 1(DE)**

## Beschreibung

Die Erfindung betrifft eine piezoelektrische Stosswellenquelle nach dem Oberbegriff des Anspruchs 1.

Aus der DE 34 25 992 - C2 ist eine piezoelektrische Stosswellenquelle mit den Merkmalen des Oberbegriffs bekannt. Die Stosswellenquelle wird nur nichtinvasiven Zerkleinerung von Konkrementen in Körpern von Lebewesen eingesetzt. Die Isolierung zwischen den Piezoelementen besteht dort (Spalte 3, Zeile 38) aus einer weichen und elektrisch isolierenden Vergußmasse.

Aufgabe der Erfindung ist es, eine Stosswellenquelle vorzuschlagen, die stärkere Stosswellen erzeugt.

Diese Aufgabe wird erfindungsgemäss mit einer piezoelektrischen Stosswellenquelle mit den Merkmalen des Anspruchs 1 gelöst.
Ausführungen der Erfindung sind Gegenstände von abhängigen Ansprüchen.

Die erfindungsgemässe flüssige oder gasförmige Isolierung ermöglicht eine wesentliche Erhöhung der elektrischen Spannungsfestigkeit. Verwendbar sind alle bekannten flüssigen und gasförmigen Isolationsmaterialien. Da diese durch ihren Aggregatzustand bedingt stets direkt an den Piezoelementen anliegen wird durch die Erfindung zuverlässig verhindert, daß - wie bei festen Isolationsmaterialien - durch die mechanische Bewegungen sich ein Luftspalt bildet, längs dessen sich die Spannung als Kriechstrom entladen kann. Erfindungsgemäß kann die Spannung - und damit die Leistung - erhöht werden. Verwendbar sind alle bekannten flüssigen Isolationsmaterialien, wie zum Beispiel Fluorcarbonate oder Oele oder die gasförmigen Isolatoren wie Schwefelhexafluorid oder Fluorcarbon.

Die erfindungsgemässe Stosswellenquelle kann mit oder ohne Membran an der Vorderseite der Piezoelemente ausgeführt sein. Wird eine Membran verwendet, so besteht sie üblicherweise aus einem Metall und dient gleichzeitig zur vorderseitigen Kontaktierung der Piezoelemente. Diese vordere Membran kann aus einem Werkstoff geeigneter akustischer Impedanz bestehen, die bei geeigneter Schichtdicke die Forderungen der Impedanzanpassung zwischen Piezokeramik und Koppelflüssigkeit (zum Beispiel Wasser oder ein Gel) erfüllt. Ein geeigneter Werkstoff ist zum Beispiel Mg Mn2 mit der Werkstoffnummer 3.5200 DIN 1729. Der Werkstoff (2) der Membran sollte die Forderung:

$$Z_2 = \sqrt{Z_1 \cdot Z_3}$$

erfüllen, wobei

$Z_2$ = akustische Impedanz
$Z_1$ = akustische Impedanz des Piezoelements
$Z_3$ = akustische Impedanz des Koppelmediums.

Die bevorzugte Schichtdicke beträgt $\lambda/4$, wobei $\lambda$ die Wellenlänge der bevorzugten Spektralkomponente des akutischen Impulses ist. Die bevorzugte Wellenlänge hänge vom Material und von der Grösse des Konkrements ab.

Möglich ist auch, dass vor jedem einzelnen Piezoelement ein geeigneter kleiner Körper zwecks Impedanzanpassung angeordnet ist.

Die elektrische Verbindung zwischen den Piezoelementen und (sofern vorhanden) der Metallmembran oder zwischen den Piezoelementen und der rückseitigen Kontaktierung (Backing), (zum Beispiel einer leitfähigen Beschichtung auf dem Träger, kann durch

a) Löten
b) Verkleben mit leitfähigem Kleber
c) Ultraschallschweißen oder
d) einfaches Anpressen

erfolgen. Bei a) und c) muss während der Herstellung weit unterhalb der Curietemperatur geblieben werden.
Die Kontaktierung kann - falls auf der entsprechenden Seite keine Membran oder Beschichtung vorliegt - auch durch Drahtbügel oder ein Geflecht oder Netz aus leitenden Elementen gebildet sein.
In einer bevorzugten Ausführung kann für das Koppelmedium und die Isolierung die gleiche Flüssigkeit verwendet werden, wodurch die vorderseitige Membran überflüssig wird und die Leistung der Quelle nochmals gesteigert wird.

Die Erfindung wird anhand dreier Figuren näher erläutert.

Die drei Figuren zeigen je Ausschnitte aus erfindungsgemässen Stosswellenquellen.

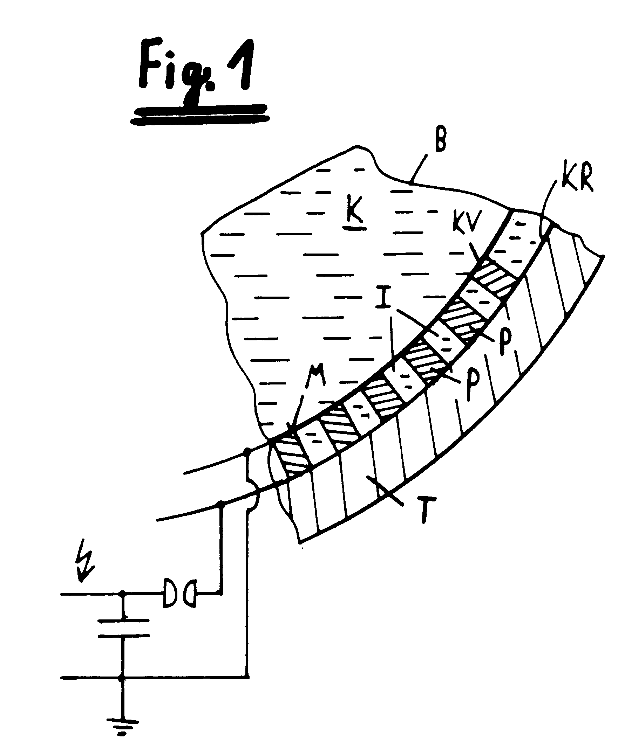

Figur 1 zeigt eine Stosswellenquelle mit dem Träger T, der hier eine Kalottenform aufweist. Auf dem Träger T sind die Piezoelemente P angeordnet. Die Piezoelemente stehen im kleinen Abstand nebeneinander und lassen Platz für die Isolierung I. An der Vorderseite und der Rückseite der Piezoelemente P sind die Kontaktierungen vorne (KV) und hinten (KB) vorgesehen. Die vordere Kontaktierung KV ist in dieser Ausführungsführung gleichzeitig als Membran M aus Metall ausgeführt. Die hintere Kontaktierung KR ist hier als leitfähige Beschichtung auf den Träger T ausgeführt. Vor der Membran M befindet sich das Koppelmedium K, das hier zum Beispiel in einem Koppelkissen B eingeschlossen ist. Das Koppelmedium K hat die Aufgabe, die Stosswellen möglichst verlustlos in den hier nicht gezeichneten Körper des Patienten einzuleiten. Die gezeigte Stosswellenquelle ist elektrisch so geschaltet, dass die Membran M auf

2

Erdpotential liegt und an die hintere Kontaktierung KR ein Hochspannungsimpuls angelegt werden kann. Dieser Spannungspuls veranlasst die Piezoelemente P zu einer kurzfristigen Kontraktion oder Ausdehnung, die diese über die Membran M an die Koppelflüssigkeit weitergeben. Dieser kurzfristige Druckpuls vereinigt sich im Brennpunkt der Kalotte zu der erwünschten Stosswelle, deren Energie ausreicht, ein Konkrement, zum Beispiel einen Nierenstein oder einen Gallenstein, zu zerkleinern. Erfindungsgemäss ist die Isolierung I eine Flüssigkeit oder ein Gas, das hier zwischen dem Träger T und der Membran M eingeschlossen ist.

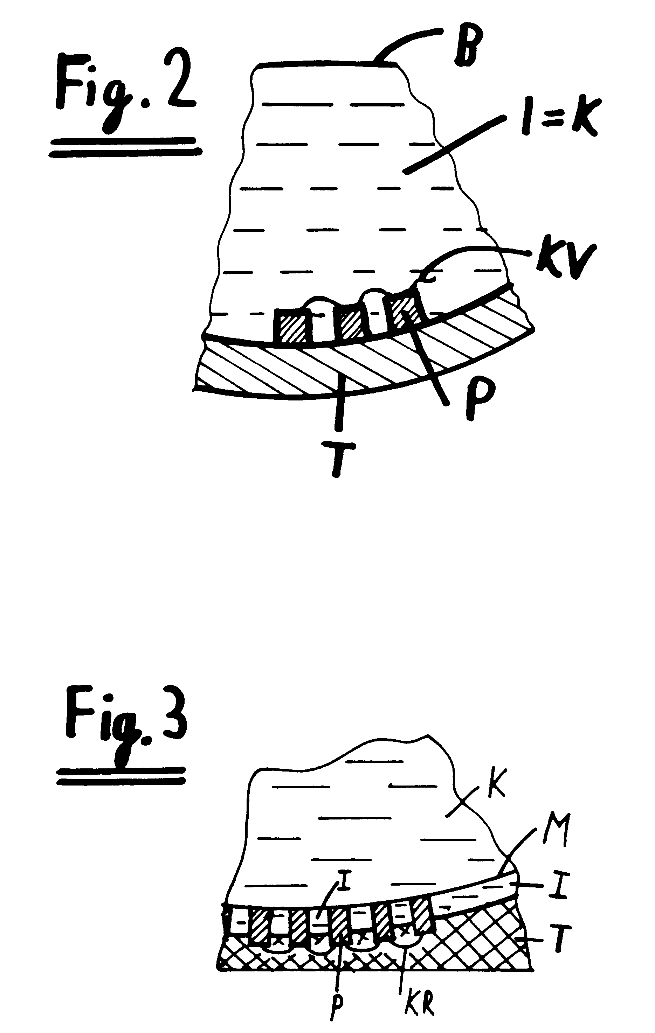

Figur 2 zeigt eine weitere Ausführung einer erfindungsgemässen Stosswellenquelle, bei der auf die geerdete Membran M verzichtet wurde. Die Piezoelemente P sind wieder auf dem Träger T angeordnet. Die vorderseitige Kontaktierung KV ist hier durch Drahtbügel realisiert. Die Isolierung I zwischen den Piezoelementen P ist gleichzeitig die Koppelflüssigkeit K, die das Koppelkissen B füllt. Als Flüssigkeit sind hier besonders Silikonöle oder Mineralöle geeignet.

Die Ausführung der Figur 2 hat durch den Wegfall der Membran M eine besonders hohe Ausgangsleistung.

Die Figur 3 zeigt eine Ausführung, bei der die rückwärtige Kontaktierung KR von Drahtbügeln oder von einem leitfähigen Geflecht übernommen wird. Die Piezoelemente sind in einem Backing oder Verguß auf der Rückseite eingegossen, der hier als Träger T fungiert. An der Vorderseite der Piezoelemente befindet sich hier eine Metallmembran M. In den Zwischenräumen zwischen dem Verguß T und der Metallmembran M befindet sich die isolierende Flüssigkeit I oder - nicht gezeigt - das isolierende Gas.

**Patentansprüche**

1.  Piezoelektrische Stosswellenquelle für medizinische Zwecke mit
    - einer Vielzahl von Piezoelementen (P)
    - einem Träger (T)
    - Kontaktierungen (KR, KV) auf einer oder beiden Seiten der Piezoelemente
    - einer Isolierung (I) zwischen den Piezoelementen (P) und
    - einem Koppelmedium (K) zum Einleiten der Stosswellen in den Körper des Patienten,
    **dadurch gekennzeichnet**, dass die Isolierung (I) flüssig oder gasförmig ist.

2.  Stosswellenquelle nach Anspruch 1, gekennzeichnet durch eine an sich bekannte Membran (M), bevorzugt aus Metall und in Form einer Kugelkalotte zwischen den Piezoelementen (P) und dem Koppelmedium (K).

3.  Stosswellenquelle nach Anspruch 2, dadurch gekennzeichnet, dass die Membran (M) impedanzmässig den Piezoelementen (P) und dem Koppelmedium (K) angepasst ist.

4.  Stosswellenquelle nach einem der vorhergehenden Ansprüche, gekennzeichnet durch einzelne Körper zur Impedanzanpassung vor den einzelnen Piezoelementen (P).

5.  Stosswellenquelle nach Anspruch 1, dadurch gekennzeichnet, dass die zur Isolierung (I) verwendete Flüssigkeit gleichzeitig das Koppelmedium (K) bildet.

6.  Stosswellenquelle nach Anspruch 1, dadurch gekennzeichnet, dass die Kontaktierungen (KR, KV) auf einer oder beiden Seiten Drahtbügel oder eine Geflecht aus leitendem Material sind.

**Claims**

1.  Piezoelectric shock wave source for medical purposes having:
    - a plurality of piezo elements (P);
    - a carrier (T);
    - bondings (KR, KV) on one or both sides of the piezo elements;
    - insulation (I) between the piezo elements (P); and
    - a coupling medium (K) for introducing the shock waves into the patient's body,
    characterized in that the insulation (I) is liquid or gaseous.

2.  Shock wave source according to claim 1, characterized by a membrane (M) which is known per se, is preferably made of metal and is in the form of a spherical segment between the piezo elements (P) and the coupling medium (K).

3.  Shock wave source according to claim 2, characterized in that the membrane (M) is adapted in terms of impedance to the piezo elements (P) and the coupling medium (K).

4.  Shock wave source according to any one of the preceding claims, characterized by individual bodies for adapting impedance in front of the individual piezo elements (P).

5.  Shock wave source according to claim 1, characterized in that the fluid used as insulation (I) is simultaneously the coupling medium (K).

**6.** Shock wave source according to claim 1, characterized in that the bondings (KR, KV) on one or both sides are wire strap or a plait of conductive material.

**Revendications**

**1.** Source d'ondes de choc piézoélectrique pour des applications médicales, comprenant une pluralité d'éléments piézoélectriques (P), un support (T), des contacts (KR, KV) sur une face ou sur les deux faces des éléments piézoélectriques, une isolation (I) entre les éléments piézoélectriques (P), et un milieu de couplage (K) pour l'introduction des ondes de choc dans le corps du patient, **caractérisée en ce** que l'isolation (I) est liquide ou gazeuse.

**2.** Source d'ondes de choc selon la revendication 1, caractérisée en ce qu'une membrane (M) bien connue en soi de préférence en métal et sous la forme d'une calotte sphérique, est disposée entre les éléments piézoélectriques (P) et le milieu de couplage (K).

**3.** Source d'ondes de choc selon la revendication 2, caractérisée en ce que l'impédance de la membrane (M) est adaptée aux éléments piézoélectriques (P) et au milieu de couplage (K).

**4.** Source d'ondes de choc selon l'une des revendications précédentes, caractérisée en ce qu'elle comprend différents corps pour l'adaptation d'impédance placés en amont des différents éléments piézoélectriques (P).

**5.** Source d'ondes de choc selon la revendication 1, caractérisée en ce que le liquide utilisé pour l'isolation (I) constitue en même temps le milieu de couplage (K).

**6.** Source d'ondes de choc selon la revendication 1, caractérisée en ce que les contacts (KR, KV) sur une face ou sur les deux faces sont constitués par des étriers de fil ou par un grillage d'un matériau conducteur.

Fig. 1

Fig. 2

B
I=K
KV
P
T

Fig. 3

K
M
I
I
T
P
KR